## Europäisches Patentamt
## European Patent Office
## Office européen des brevets

(19)

(11) Numéro de publication: **0 276 601**

**A1**

(12) # DEMANDE DE BREVET EUROPEEN

(21) Numéro de dépôt: 87402876.4

(22) Date de dépôt: **16.12.87**

(51) Int. Cl.⁴: **A61B 17/22** , A61B 8/08

(30) Priorité: **31.12.86 FR 8618441**

(43) Date de publication de la demande:
**03.08.88 Bulletin 88/31**

(84) Etats contractants désignés:
**AT BE CH DE ES FR GB GR IT LI LU NL SE**

(71) Demandeur: **TECHNOMED INTERNATIONAL S.A.**
**28, rue Desaix**
**F-75015 Paris(FR)**

Demandeur: **INSERM INSTITUT NATIONAL DE LA SANTE ET DE LA RECHERCHE MEDICALE**
**151, Cours Albert Thomas**
**F-69003 Lyon(FR)**

(72) Inventeur: **Mestas, Jean-Louis**
**1, Impasse Marc Séguin**
**F-69680 Chassieu(FR)**
Inventeur: **Lacruche, Bernard**
**29, rue Alphonse Bordereau**
**F-77500 Chelles(FR)**
Inventeur: **Cathignol, Dominique**
**14, rue du Fort**
**F-69740 Genas(FR)**

(74) Mandataire: **Portal, Gérard et al**
**Cabinet Beau de Loménie 55, rue d'Amsterdam**
**F-75008 Paris(FR)**

(54) **Procédé et dispositif de coordination des positions initiales dans l'espace d'un dispositif de déclenchement et de concentration de tirs sur un point cible et d'un bras de repérage de la position de cette cible.**

(57) L'invention concerne un procédé et un dispositif de coordination des positions initiales dans l'espace d'un dispositif de déclenchement et de concentration des tirs (10) avec un bras de repérage (14).

Ce dispositif de coordination est caractérisé en ce qu'il comprend des moyens (20) de calibrage de distance (D) entre l'extrémité libre (14a) du bras de repérage (14) et le dispositif de déclenchement et de concentration des tirs (10), qui comprennent de préférence un organe de liaison (22).

On améliore ainsi la précision du repérage et l'efficacité de destruction des cibles.

Fig.1

## Procédé et dispositif de coordination des positions initiales dans l'espace d'un dispositif de déclenchement et de concentration de tirs sur un point cible et d'un bras de repérage de la position de cette cible.

L'invention concerne essentiellement un procédé et dispositif de coordination des positions initiales dans l'espace d'un dispositif de déclenchement et de concentration de tirs sur un point cible et d'un bras de repérage de la position de cette cible.

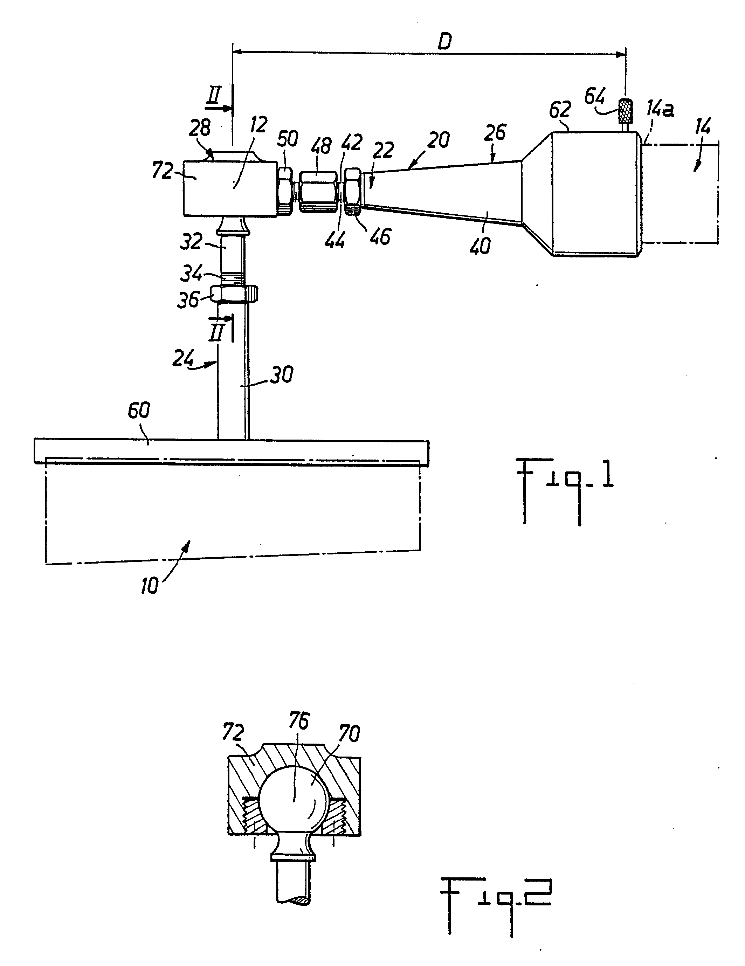

Il est connu par le brevet US Rieber n° 2 559 227 un dispositif de déclenchement et de concentration de tirs sur un point cible destiné à venir en coïncidence avec une cible à détruire, par exemple des tissus d'un être vivant, ce dispositif peut également être utilisé pour détruire des lithiases. Ce dispositif peut comprendre un réflecteur ellipsoïdal tronqué 80 dans lequel sont générées des ondes de choc par décharge ou arc électrique entre deux électrodes 12, 13 agencées pour être concourantes au foyer interne de l'ellipsoïde qui est rempli d'un liquide de transmission des ondes de choc 83, constitué par exemple par une huile (voir figure 3 et colonne 7, ligne 51 colonne 9, ligne 30).

De même, le document FR-A-2 240 795 décrit un appareil similaire dans lequel le liquide est constitué par de l'eau (page 3, lignes 23-24).

On connaît par ailleurs par le document FR-A-2 502 485 un bras 18, 20 de repérage supportant à son extrémité libre une sonde 10 de repérage du type à ultrasons. Ce bras de repérage comprend en particulier 6 articulations qui sont chacune pourvues d'un détecteur d'angle de rotation 26, 28, 30, 32, 34, 36 afin d'établir un diagnostic en affichant un tomogramme de l'échantillon examiné.

On connaît encore par le document EP-169 311 un bras de repérage supportant à son extrémité libre une sonde de repérage de type à rayons X ou à ultrasons, qui est montée sur un appareil de déclenchement et de concentration de tir sur un point cible destiné à venir en coïncidence avec une cible à détruire, par exemple une lithiase. Ce dispositif de déclenchement et de concentration de tir est constitué par un réflecteur ellipsoïdal tronqué, les tirs étant constitués par des ondes de choc générées au premier foyer de l'ellipsoïde qui concentre ces tirs sur le second foyer, comme décrit dans Rieber US 2 559 227.

Ainsi, la difficulté réside dans le fait de faire coïncider la cible repérée par la sonde de repérage située à l'extrémité du bras de repérage avec le second foyer de l'ellipsoïde constituant un point cible, cet ellipsoïde étant monté sur un support qui est déplaçable dans les trois directions de l'espace X, Y, Z. Les présents inventeurs ont trouvé qu'il est nécessaire de connaître la position initiale, dite de référence, dans l'espace du dispositif de déclenchement et de concentration de tir, par exemple un réflecteur ellipsoïdal, et de coordonner cette position initiale, de référence, avec la position initiale, de référence, du bras de repérage.

Dans ce document EP-1 693 111, on ne décrit ni ne suggère rien de tel.

Ainsi, la présente invention a pour but de résoudre un nouveau problème technique consistant en la fourniture d'une solution permettant de coordonner la position initiale, dite de référence, dans l'espace d'un dispositif de déclenchement et de concentration de tir sur un point cible destiné à venir en coïncidence avec une cible à détruire, par exemple une lithiase, avec la position initiale, dite de référence, d'un bras de repérage de la position de cette cible, en augmentant ainsi de manière significative la précision de repérage et l'amenée en coïncidence de la cible repérée par la sonde de repérage disposée à l'extrémité du bras de repérage avec le point cible déterminé par le dispositif de déclenchement et de concentration de tirs, en augmentant ainsi de manière inattendue, l'efficacité de destruction des cibles.

La présente invention a encore pour but principal de résoudre le nouveau problème technique consistant en la fourniture d'une solution permettant de réaliser la coordination des positions initiales, dites de référence, dans l'espace, du dispositif de déclenchement et de concentration de tir sur un point cible destiné à venir en coïncidence avec une cible à détruire, par exemple une lithiase, et d'un bras de repérage de la position de cette cible, supportant à son extrémité libre une sonde de repérage, et permettant de détecter des imperfections dans les déplacements dans l'espace de ce dispositif de déclenchementet de concentration de tir et du bras de repérage, en permettant ainsi de corriger ces imperfections, en améliorant ainsi, de manière inattendue, l'exactitude des repérages dans l'espace et l'amenée ultérieure en coïncidence de la cible à détruire avec le point cible, en conduisant encore à une amélioration concomitante de l'efficacité de destruction des cibles.

Ces nouveaux problèmes techniques sont résolus pour la première fois par la présente invention par la fourniture d'une solution d'un coût négligeable mais augmentant le nombre de cibles détruites tels que des tissus, concrétions, comme des lithiases rénales, biliaires, etc., et diminuant la durée des séances de tirs, par l'amélioration de la précision de repérage de la cible et de l'amenée en coïncidence de cette cible avec le point cible.

Ainsi, selon un premier aspect, la présente invention fournit un procédé de coordination de la position initiale, dite de référence, dans l'espace d'un dispositif de déclenchement et de concentration de tir sur un point cible destiné à venir en coïncidence avec une cible à détruire, par exemple une lithiase, avec la position initiale, dite de référence, d'un bras de repérage de la position initiale de ladite cible, supportant à son extrémité libre une sonde de repérage, par exemple du type à ultrasons, pour permettre d'amener la cible en coïncidence avec le point cible, caractérisé en ce qu'on calibre la distance entre l'extrémité libre du bras et le dispositif de déclenchement et de concentration des tirs.

Selon une caractéristique préférée, on calibre la distance entre l'extrémité libre du bras et le point cible.

Selon une autre caractéristique avantageuse du procédé selon l'invention, on réalise le calibrage en incorporant un organe de liaison entre le dispositif de déclenchement et de concentration des tirs et l'extrémité libre du bras.

Selon une autre caractéristique avantageuse du procédé selon l'invention, l'organe de liaison précité comprend un premier élément de liaison et un second élément de liaison articulés entre eux par une articulation permettant avantageusement un déplacement dans les trois directions de l'espace, de préférence du type rotule, un élément de liaison étant monté sur le dispositif de déclenchement et de concentration des tires, l'autre élément de liaison étant monté à l'extrémité libre du bras en lieu et place de la sonde de repérage.

Selon une autre caractéristique préférée de l'invention, chaque élément de liaison est de longueur réglable ainsi, selon le procédé de l'invention, on fait coïncider l'axe ou point d'articulation desdits éléments de liaison avec le point cible en réglant la longueur de l'élément de liaison monté sur le dispositif de déclenchement et de concentration des tirs, et on règle ensuite la distance entre l'extrémité du bras et le point cible à une valeur de distance de référence, en agissant sur la longueur de l'élément de liaison monté sur le bras de repérage.

Avantageusement, cette distance dê référence est au minimum égale à la plus grande distance de détection de profondeur de la sonde de repérage montée sur le bras.

Selon encore une autre caractéristique du procédé selon l'invention, on déplace le dispositif de déclenchement et de concentration des tirs, successivement selon les trois axes X, Y, Z d'une distance prédéterminée, pour détecter des imperfections dans le déplacement dans l'espace du bras de repérage et du dispositif de déclenchement et de concentration des tirs.

L'invention concerne aussi, selon un deuxième aspect, un dispositif de coordination de la position initiale, dite de référence, dans l'espace d'un dispositif de déclenchement et de concentration de tirs sur un point cible destiné à venir en coïncidence avec une cible à détruire, par exemple une lithiase, avec la position initiale, dite de référence, d'un bras de repérage de la position de ladite cible, supportant à son extrémité libre une sonde de repérage, par exemple du type à ultrasons, pour permettre d'amener la cible en coïncidence avec le point cible, caractérisé en ce qu'il comprend des moyens de calibrage de la distance entre l'extrémité libre dudit bras et le dispositif de déclenchement et de concentration des tirs. De préférence, ces moyens de calibrage sont des moyens de calibrage entre l'extrémité libre du bras et le point cible.

Selon encore une caractéristique préférée de l'invention, les moyens de calibrage précités comprennent un organe de liaison entre le dispositif de déclenchement et de concentration des tirs et l'extrémité libre du bras.

Cet organe de liaison peut être défini comme précédemment.

Selon une autre caractéristique particulièrement avantageuse de l'invention, le dispositif de déclenchement et de concentration des tirs est constitué par un réflecteur ellipsoïdal tronqué, du type décrit dans le brevet US Rieber n° 2 559 227 ou dans le document FR-A-2 247 195.

On comprend ainsi que l'invention permet de coordonner d'une manière extrêmement simple et pratique, les positions initiales, de référence, dans l'espace du dispositif de déclenchement et de concentration des tirs avec le bras de repérage, ce qui permet d'améliorer l'exactitude des repérages, et ainsi la précision de l'amenée en coïncidence de la cible à détruire, repérée par la sonde de repérage disposée à l'extrémité libre du bras de repérage, avec le point cible. Ainsi, on aboutit à une amélioration significative de l'efficacité de destruction des cibles, ce qui conduit à son tour à une limitation du nombre des tirs et de la durée des traitements, ce qui permet en pratique d'augmenter le nombre de patients traités.

D'autres buts, caractéristiques et avantages de l'invention apparaîtront clairement à la lumière de la description explicative qui va suivre faite en référence aux dessins annexés, représentant un mode de réalisation actuellement préféré de l'invention donné simplement à titre d'illustration et qui ne saurait donc en aucune façon limiter la portée de l'invention. Dans les dessins :

- la figure 1 est une vue partielle, en élévation, du mode actuellement préféré du dispositif de coordination selon l'invention, en position montée entre le dispositif de déclenchement et de concentration des tirs, ici constitué par un réflecteur ellipsoïdal tronqué, et un bras de repérage ; et

- la figure 2 représente une vue en coupe selon la ligne de trace II-II de la figure 1.

En référence aux figures 1 et 2, un dispositif selon l'invention de coordination de la position initiale, dite de référence dans l'espace d'un dispositif de déclenchement et de concentration des tirs, représenté par le numéro de référence général 10, sur un point cible 12 destiné à venir en coïncidence avec une cible à détruire, par exemple une lithiase, avec la position initiale, dite de référence, d'un bras de repérage, représenté par le numéro de référence général 14, supportant à son extrémité libre une sonde de repérage, par exemple du type à ultrasons, pour permettre d'amener la cible en coïncidence avec le point cible 12, est caractérisé en ce qu'il comprend des moyens de calibrage, représentés par le numéro de référence général 20, de la distance D entre l'extrémité libre 14a, du bras 14 et le dispositif de déclenchement et de concentration des tirs 10.

De préférence, ces moyens de calibrage 20 sont des moyens de calibrage entre l'extrémité libre 14a du bras 14 et le point cible 12.

Selon le mode de réalisation actuellement préféré, ces moyens de calibrage 20 comprennent un organe de liaison représenté par le numéro de référence général 22 assurant une liaison, ici mécanique, entre le dispositif de déclenchement et de concentration des tirs 10 et l'extrémité libre 14a du bras de repérage 14.

Selon le mode de réalisation représenté, cet organe de liaison 22 comprend avantageusement un premier élément de liaison 24 et un second élément de liaison 26 articulés entre eux par une articulation 28, permettant avantageusement un déplacement dans les trois directions de l'espace, de préférence du type à rotule, comme représenté. Ainsi, un élément de liaison, par exemple le premier élément de liaison 24, est monté sur le dispositif de déclenchement et de concentration des tirs 10 et l'autre élément de liaison, ici le second élément de liaison 26 est monté à l'extrémité libre 14a du bras de repérage 14 en lieu et place de la sonde de repérage.

Selon une autre caractéristique préférée de l'invention, chaque élément de liaison 24, 26 est de longueur réglable en étant réalisé de manière téléscopique. Ainsi, le premier élément de liaison 24 comprend en fait une partie fixe 30 dans laquelle est montée de manière déplaçable une partie mobile ou téléscopique 32, dont le déplacement peut être réglé à volonté et par exemple, par un système vis 34 - écrou 36.

De même, le deuxième élément de liaison 26 comprend une partie fixe 40 et une partie téléscopique 42 déplaçables au moins en partie dans ou hors de la partie fixe 40 par un système téléscopique classique, par exemple du type vis 44 - écrou 46. On peut prévoir plusieurs écrous, 46, 48, 50 de manière à accroître la précision de réglage. Ceci peut également être réalisé sur chaque élément de liaison 24, 26.

On comprend aisément que le premier élément de liaison 24, qui est ici destiné à être monté sur le dispositif de déclenchement et de concentration des tirs comprend des moyens d'adaptation à ce dispositif correspondants.Ici, étant donné que le dispositif de déclenchement et de concentration des tirs est par exemple constitué par un réflecteur ellipsoïdal tronqué, la partie fixe 30 du premier élément de liaison 24 est pourvue d'une platine 60 emmanchée sur la partie tronquée du réflecteur ellipsoïdal 10. De même, la partie fixe 40 du second élément de liaison 26 comprend des moyens d'adaptation 62 venant s'emmancher dans l'extrémité libre 14a du bras 14, en pouvant y être verrouillés par un élément de verrouillage 64 tel qu'une vis.

D'autre part, l'articulation 28 permettant un déplacement dans les trois directions de l'espace, est de préférence du type à rotule, comme cela est clairement visible à la figure 2. Ainsi, par exemple la partie déplaçable téléscopique 32 du premier élément 24 se termine par une partie en forme de rotule hémisphérique 70 sur laquelle est disposée la deuxième partie de l'articulation constituée par une calotte correspondante également hémisphérique 72 qui est solidaire de la partie déplaçable ou téléscopique 42 du deuxième élément de liaison 26. On conçoit ainsi que ce dispositif de coordination permet de réaliser le procédé de coordination précédemment décrit et qui est le suivant.

Tout d'abord, on emmanche donc la platine 60 sur le dispositif de déclenchement et de concentration des tirs 10, ici un réflecteur ellipsoïdal tronqué, puis on emmanche la partie 62 du deuxième élément de liaison 26 à l'extrémité libre 14a du bras de repérage 14.

On comprend que le dispositif de déclenchement et de concentration des tirs 10 monté sur un support déplaçable dans les trois directions de l'espace permette un déplacement de celui-ci dans une position quelconque de l'espace, ce support étant par exemple décrit dans le document FR-8 609 474. Il en est de même du bras de repérage dont l'extrémité libre 14a peut être déplacée dans une position quelconque de l'espace, notamment en étant conçue pour présenter six numéros de liberté dans la présence de six articulations, comme par exemple décrits dans le document FR-2 502 485 ou dans le document FR-86 06 319.

Ainsi, pour réaliser la coordination des positions initiales, on comprend que le dispositif de déclenchement et de concentration des tirs est tout d'abord mis dans une position initiale pour laquelle le dispositif de déclenchement et de concentration des tirs est sensiblement au voisinage de, où se trouve à, la position zéro, suivant l'axe X, selon l'axe Y, et selon l'axe Z. Dans cette position initiale, on règle alors la longueur du premier élément de liaison 24 de l'organe de liaison 22, en agissant sur l'écrou de réglage 36, de manière à amener l'axe ou point d'articulation de l'articulation 28, qui est ici constitué par le centre 76 de la rotule hémisphérique 70 qui coïncide avec celui de la calotte hémisphérique 72 ; en coïncidence avec le point cible 12, qui est ici défini par le second foyer de l'ellipsoïde tronquée 10.

Ensuite, on règle la longueur du deuxième élément de liaison 26 pour que l'extrémité 14a du bras 14 se trouve à une distance prédéterminée D du point cible 12, ce qui permet de calibrer la distance entre l'extrémité libre 14a du bras de repérage 14 et le dispositif de déclenchement et de concentration des tirs, plus précisément le point cible 12.

Cette distance D prédéterminée est de préférence au minimum égale à la plus grande distance de détection en profondeur de la sonde de repérage utilisée, qui sera montée à l'extrémité libre 14a du bras 14. On comprend que pour régler la longueur du second élément de liaison 26 monté à l'extrémité libre 14a du bras 14, on peut agir aisément sur les écrous 46, 48, 50.

Grâce au moyen de coordination 20, comprenant l'organe de liaison 22, on conçoit ainsi que l'on peut coordonner les positions initiales dans l'espace du dispositif de déclenchement et de concentration des tirs 10 et du bras de repérage 14. Les coordonnées de cette position initiale du bras de repérage sont relevées et elles seront ensuite utilisées pour déterminer la position dans l'espace d'une cible à détruire, par exemple une lithiase, par la position occupée à ce moment là dans l'espace de ce bras de repérage, ce qui permettra de modifier la position dans l'espace du dispositif de déclenchement et de concentration des tirs 10 pour ramener le point cible 12 en coïncidence avec la cible à détruire.

On comprend ainsi que l'invention fournit un procédé et un dispositif de coordination des positions initiales dans l'espace extrêmement simple, et extrêmement aisé à manipuler, permettant d'effectuer un calibrage extrêmement précis. Par ailleurs, ceci constitue encore une caractéristique particulièrement avantageuse de l'invention, l'organe de liaison 22 étant monté comme représenté à la figure 1, après avoir effectué le calibrage comme décrit ci-dessus, on déplace le dispositif de déclenchement et de concentration des tirs 10 successivement selon les trois axes X, Y, Z, d'une distance prédéterminée, le bras de repérage étant entraîné grâce à l'organe de liaison 22, et on détecte les nouvelles coordonnées dans l'espace de l'extrémité libre 14a du bras 14.

Ceci permet de détecter des imperfections dans le déplacement dans l'espace du dispositif de déclenchement et de concentration des tirs 10 et/ou du bras de repérage 14.

En effet, si les coordonnées relevées pour l'extrémité libre 14a du bras 14 sont différentes de celles qu'elles devraient être en fonction du déplacement prédéterminé du dispositif de déclenchement et de concentration des tirs 10, alors il existe une imperfection dans le déplacement dans l'espace soit du dispositif de déclenchement et de concentration des tirs 10, soit du bras de repérage 14, soit des deux.

On corrige alors ces imperfections, qui sont dues à des imperfections mécaniques ou du support du dispositif de déclenchement et de concentration des tirs 10 ou du bras de repérage 14.

Ainsi, l'invention permet encore, d'une manière inattendue, de corriger des imperfections de fabrication soit du bras de repérage 14, soit du support du dispositif de déclenchement et de concentration des tirs 10.

Naturellement, l'invention comprend tous les moyens constituant des équivalents techniques des moyens décrits ainsi que leurs diverses combinaisons et on comprend ainsi que l'on peut utiliser tout type de dispositif de déclenchement et de concentration des tirs 10 sur un point cible 12, ainsi que tout type de bras de repérage capable d'être déplacé dans une position quelconque de l'espace.

## Revendications

1. Procédé de coordination de la position initiale, dite de référence, dans l'espace d'un dispositif de déclenchement et de concentration de tirs (10) sur un point cible (12), destiné à venir en coïncidence avec une cible à détruire, par exemple une lithiase, avec la position initiale, dite de référence, d'un bras de repérage (14) de la position de ladite cible, supportant à son extrémité libre (14a) une sonde de repérage, par exemple du type à ultrasons, pour permettre d'amener la cible en coïncidence avec le point cible (12), caractérisé en ce qu'on calibre la distance (D) entre l'extrémité libre du bras et le dispositif de déclenchement et de concentration des tirs (10).

2. Procédé selon la revendication 1, caractérisé en ce qu'on calibre la distance (D) entre l'extrémité libre du bras (14) et le point cible (12).

3. Procédé selon la revendication 1 ou 2, caractérisé en ce qu'on réalise le calibrage en incorporant un organe de liaison (22) entre le dispositif de déclenchement et de concentration des tirs (10) et l'extrémité libre (14a) du bras (14).

4. Procédé selon la revendication 3, caractérisé en ce que l'organe de liaison (22) comprend un premier élément de liaison (24) et un second élément de liaison (26) articulés entre eux par une articulation (28) permettant avantageusement un déplacement dans les trois directions de l'espace, de préférence du type à rotule, un élément de liaison (24) étant monté sur le dispositif de déclenchement et de concentration des tirs (10) et l'autre élément de liaison (26) étant monté sur l'extrémité libre (14a) du bras de repérage (14) en lieu et place de la sonde de repérage.

5. Procédé selon la revendication 4, caractérisé en ce que chaque élément de liaison (24, 26) est de longueur réglable ; on fait coïncider l'axe ou point d'articulation (76) desdits éléments de liaison (24, 26) avec le point cible (12) en réglant la longueur de l'élément de liaison (24) monté sur le dispositif de déclenchement et de concentration des tirs (10) ; et on règle ensuite la distance (D) entre l'extrémité libre (14a) du bras (14) et le point cible (12) à une valeur de distance de référence, en agissant sur la longueur de l'élément de liaison (26) monté sur le bras (14).

6. Procédé selon la revendication 5, caractérisé en ce que cette distance de référence est au minimum égale à la plus grande distance de détection en profondeur de la sonde de repérage.

7. Procédé selon l'une quelconque des revendications 1 5, caractérisé en ce qu'on déplace le dispositif de déclenchement et de concentration des tirs (10) successivement selon les trois axes X, Y, Z, d'une distance prédéterminée pour détecter des imperfections dans le déplacement dans l'espace du bras de repérage (14) et/ou du dispositif de déclenchement et de concentration des tirs (10).

8. Dispositif de coordination de la position initiale, dite de référence, dans l'espace d'un dispositif de déclenchement et de concentration des tirs (10), sur un point cible (12), destiné à venir en coïncidence avec une cible à détruire, par exemple une lithiase, avec la position initiale, dite de référence, d'un bras de repérage (14) de la position de ladite cible, supportant à son extrémité libre (14a) une sonde de repérage, par exemple du type à ultrasons, pour permettre d'amener la cible en coïncidence avec le point cible (12), caractérisé en ce qu'il comprend des moyens de calibrage (20) de la distance (D) entre l'extrémité libre (14a) dudit bras (14) et le dispositif de déclenchement et de concentration des tirs (10).

9. Dispositif selon la revendication 8, caractérisé en ce que les moyens de calibrage précité (20) sont des moyens de calibrage entre l'extrémité libre (14a) du bras (14) et le point cible (12).

10. Dispositif selon la revendication 8 ou 9, caractérisé en ce que les moyens de calibrage (20) comprennent un organe de liaison (22) entre le dispositif de déclenchement et de concentration des tirs (10) et l'extrémité libre (14a) du bras (14).

11. Dispositif selon la revendication 10, caractérisé en ce que l'organe de liaison (22) comprend un premier élément (24) et un second élément de liaison (26) articulés entre eux par une articulation (28) permettant avantageusement un déplacement dans les trois directions de l'espace, de préférence du type à rotule, un élément de liaison (24) étant monté sur le dispositif de déclenchement et de concentration des tirs (10) et l'autre élément de liaison (26) étant monté à l'extrémité libre (14a) du bras de repérage (14) en lieu et place de la sonde de repérage.

12. Dispositif selon la revendication 11, caractérisé en ce que chaque élément de liaison (24, 26) est de longueur réglable.

13. Dispositif selon l'une quelconque des revendications 8 à 12, caractérisé en ce que le dispositif de déclenchement et de concentration des tirs (10) est constitué par un réflecteur ellipsoïdal tronqué.

Fig. 1

Fig. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | FR-A-2 502 485  (ALOKA CO. LTD)<br>* Revendication; page 4, ligne 16 – page 5, ligne 28; figures 1,2 *<br>--- | | A 61 B   17/22<br>A 61 B   8/08 |
| A,D | EP-A-0 169 311  (DORNIER GmbH)<br>* Revendications; figures 1,2 *<br>--- | | |
| A | DE-U-8 528 785  (SIEMENS AG)<br>* Revendication 1; figures 1-4 *<br>--- | | |
| A | DE-U-8 515 656  (SIEMENS AG)<br>* En entier *<br>----- | | |

DOMAINES TECHNIQUES
RECHERCHES (Int. Cl.4)

A 61 B
G 10 K
G 01 S

Le présent rapport a été établi pour toutes les revendications

| Lieu de la recherche | Date d'achèvement de la recherche | Examinateur |
|---|---|---|
| LA HAYE | 07-04-1988 | NEILL M.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................
& : membre de la même famille, document correspondant

EPO FORM 1503 03.82 (P0402)